(19)

Europäisches Patentamt

European Patent Office

Office européen des brevets

(11) **EP 3 028 629 A1**

(12) **EUROPEAN PATENT APPLICATION**

(43) Date of publication:
**08.06.2016 Bulletin 2016/23**

(51) Int Cl.:
*A61B 5/024* ^(2006.01)  *A61B 5/00* ^(2006.01)

(21) Application number: **15151890.9**

(22) Date of filing: **21.01.2015**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **01.12.2014 EP 14195689**

(71) Applicants:
• **IMEC VZW**
  **3001 Leuven (BE)**

• **Katholieke Universiteit Leuven**
  **3000 Leuven (BE)**

(72) Inventor: **Pamula, Venkata Rajesh**
  **3001 Leuven (BE)**

(74) Representative: **Patent Department IMEC**
  **IMEC vzw**
  **Patent Department**
  **Kapeldreef 75**
  **3001 Leuven (BE)**

(54) **System and method for heart rate detection**

(57)    The invention relates to a system (100) for heart rate detection comprising a random sampling module (10) configured for providing nonuniform random samples (S1) below Nyquist rate of a biosignal that contains heart rate information; and a heart rate detection module (30) configured for receiving a plurality of said nonuniform random samples (S1) during a predetermined time window, calculate a power spectral density based on a Lomb-Scargle periodogram of said window samples and calculate a heart rate value (S2) based on the frequency corresponding to the highest power peak of the calculated power spectral density. The invention also relates to a corresponding method for heart rate detection and computer program product.

# Figure 1

**100**

EP 3 028 629 A1

**Description**

**Technical Field**

The present description relates generally to biosignal acquisition systems and more specifically to systems and methods for heart rate and/or heart rate variability detection with randomly sampled biosignals.

**Background**

Compressed sensing or compressive sampling (CS) is an emerging signal processing technique that asserts that certain signals can be recovered faithfully from far fewer number of samples or measurements. CS relies on the underlying structure of the signal which is expressed terms of its *'sparsity'* and the *'incoherence'* which is related to the sampling scheme (see for example "An Introduction to compressive sampling", E. J. Candès et al., IEEE Signal Processing Magazine, vol. 25, pp 21-30, Mar. 2008).

Known state-of-the-art biosignal acquisition systems using, for example, the techniques described in "Compressed Sensing System Considerations for ECG and EMG Wireless Biosensors", A.M.R. Dixon et al., IEEE Transactions on Biomedical Circuits and Systems, vol. 6, No.2, April 2012, require, for the detection of a specific biosignal feature, such as for example the heart rate (HR), to first reconstruct an approximation of the original sampled biosignal. This means that complex signal reconstruction algorithms have to be run on the received samples in order to obtain a time domain reconstructed signal and then perform known feature extraction techniques, such as HR or heart rate variability (HRV) detection, on that time domain signal. Such reconstruction process and detection techniques are computationally intensive and hence not suited for low power sensor nodes. Typically, as described in "Artifacts Mitigation in Ambulatory ECG Telemetry", H. Garudari et al., Proc. IEEE Int. Conf. e-Health Networking Applications and Services, pp.338 -344, 2010, the reconstruction complex processing is offloaded from the sensor and performed at a separated receiver node or base station. With this technique the sensor can work with low power budget while the receiver node, which is assumed to have a better battery budget or no restrictions on power consumption, performs the computationally intensive tasks. Another exemplary system describing a HR detector using CS techniques is described in paper "An ultra low power pulse oximeter sensor based on compressed sensing", P.K. Baheti et al., Body Sensor Networks 2009, pp 144-148, Berkeley, USA 2009.

There is a motivation to improve current state of the art systems and methods in order to reduce HR and/or HRV detection complexity and/or power consumption.

**Summary**

The present invention provides a new and improved system and method for HR and/or HRV detection from a randomly sampled biosignal that contains HR information, such as for example, an electrocardiogram (ECG), a ballistocardiogram (BCG) or a photoplethysmogram (PPG) signal.

According to an exemplary embodiment, the system and method for HR and/or HRV detection can estimate the HR and/or the HRV from the received measurements of a randomly sampled ECG, BCG or PPG biosignal without having to reconstruct the original time domain signal.

According to an exemplary embodiment, the system and method for HR and/or HRV detection advantageously reduces HR and/or HRV estimation complexity and/or power consumption compared to state of the art CS techniques.

According to an exemplary embodiment, the system and method for HR and/or HRV detection advantageously reduces the number of samples of the sampled biosignal needed for faithful HR and/or HRV extraction compared to state of the art CS techniques.

According to an exemplary embodiment, the system and method for HR and/or HRV detection can advantageously be implemented at the sensor node without significantly increasing the power consumption.

According to an exemplary embodiment, the system is a low power and computationally efficient ECG, BCG or pulseoximetry system capable of estimating HR and/or HRV on the sensor node without having to reconstruct the compressively or randomly sampled data.

According to an exemplary embodiment, there is provided a system for heart rate detection comprising: a random sampling module configured for providing nonuniform random samples below Nyquist rate of a biosignal that contains heart rate information; and a heart rate detection module configured for receiving a plurality of said nonuniform random samples during a predetermined time window and calculating a heart rate value based on the received time window samples; wherein the heart rate detection module is configured to calculate a power spectral density based on a Lomb-Scargle periodogram of said window samples and calculate the heart rate value based on the frequency corresponding to the highest power peak of the calculated power spectral density.

According to an exemplary embodiment, the heart rate detection module may be configured to allocate the received nonuniform random samples into a plurality of windows of samples and calculate the heart rate value for that plurality of window samples.

According to an exemplary embodiment, the heart rate detection module may be configured to search for the highest power peak of the calculated power spectral density of a window of samples within a limited frequency range around an average highest power peak frequency calculated based on a plurality of windows.

According to an exemplary embodiment, the heart rate detection module may be configured to calculate the power spectral density of a window of samples with the use of data stored in a look-up table, the data representing a trigonometric calculation.

According to an exemplary embodiment, the heart rate detection module may be configured to calculate the power spectral density of a window of samples in a frequency band between 0.5 and 5Hz and with a frequency resolution of 0.08 Hz.

According to an exemplary embodiment, the time interval or window of samples is at least 4 seconds.

According to an exemplary embodiment, heart rate detection module may be further configured to calculate a heart rate variability value as the difference between the heart rate value of two consecutive windows of samples.

There is also provided an electronic device comprising an ECG, BCG or Pulseoximetry system for heart rate detection according to any of the exemplary embodiments herein described.

There is also provided a method for heart rate detection comprising: receiving a plurality of nonuniform random samples below Nyquist rate of a biosignal that contains heart rate information; calculating a power spectral density based on a Lomb-Scargle periodogram of said received plurality of samples; finding a frequency corresponding to the highest power peak of the calculated power spectral density; and calculating a heart rate value based on said found highest peak power frequency.

Accoring to an exemplary embodiment, the method may further comprise: allocating the plurality of received nonuniform random samples into a plurality of windows of samples and calculating the heart rate value for at least two consecutive windows of samples; and calculating a heart rate variability value as the difference between the heart rate value of said two consecutive windows of samples.

There is also provided a computer program product and a computer readable storage medium according to embodiments of the invention.

## Brief description of the drawings

The above and other aspects of the system and method according to the present description will be shown and explained with reference to the non-restrictive example embodiments described hereinafter.

**Figure 1** shows a general block diagram of an exemplary system for heart rate detection according to the present invention.

**Figures 2A and 2B** show, respectively, exemplary graphs of four time domain PPG signal windows and the corresponding four PSD calculations when said time domain PPG signal windows are randomly sampled at an average sampling rate of 12 Hz, corresponding to a Compression Ratio of 10, according to an exemplary embodiment of the invention.

**Figures 3A and 3B** show, respectively, an exemplary graph of a time domain PPG signal window, which is randomly sampled at an average sampling rate of 4 Hz, corresponding to a Compression Ratio of 30 and the corresponding PSD calculation.

**Figure 4** shows an exemplary graph of the HRV value calculated using a method according to an embodiment the invention and a conventional time domain method.

**Figure 5** shows a flow diagram of a method for calculating HR according to an exemplary embodiment of the invention.

**Figure 6** shows a flow diagram of a method for calculating HRV according to an exemplary embodiment of the invention.

## Detailed description

In the following, in the description of exemplary embodiments, various features may be grouped together in a single embodiment, figure, or description thereof for the purpose of streamlining the disclosure and aiding in the understanding of one or more of the various inventive aspects. This is however not to be interpreted as the invention requiring more features than the ones expressly recited in the main claim. Furthermore, combinations of features of different embodiments are meant to be within the scope of the invention, as would be clearly understood by those skilled in the art. Additionally, in other instances, well-known methods, structures and techniques have not been shown in detail in order not to obscure the conciseness of the description.

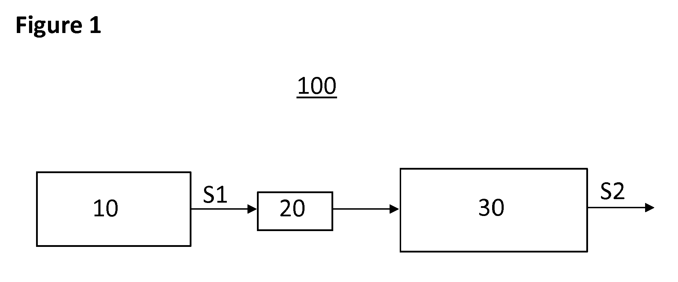

**Figure 1** shows a general block diagram of an exemplary system for heart rate detection **100** comprising a random sampling module **10** providing a randomly sampled biosignal **S1,** a signal conditioning module **20,** and a HR detection module **30** providing information about a subject's HR and/or HRV **S2.** The random sampling module **10** may include a sensor that generates an analogue version of a certain sensed biosignal, e.g. ECG, BCG or PPG and said signal is then sampled according to current state of the art nonuniform random sampling techniques (below Nyquist sampling rate) so that a random-sampled version **S1** of the sensed biosignal is provided to the next signal conditioning, transmission and/or processing stages. References for nonuniform random sampling techniques can be found in "Compressive Sensing by Random Convolution", by J. Romberg, SIAM Journal on Imaging Sciences, vol.2, no.4, Oct 2009; and "Sparsity and Incoherence in Compressive Sampling", by E. Candes and J. Romberg, Inverse Prob., vol. 23, no. 3, pp. 969-985, 2007. Alternatively, the random sampling module **10** may trigger or activate a sensor according to current state of the art nonuniform random sampling techniques (below Nyquist sampling rate) so that said sensor directly generates a random sampled version **S1** of the sensed biosignal. The signal conditioning module **20** may be optional and comprise an analogue to digital converter and/or any other element for conditioning of the randomly sampled signal **S1** to a transmission means. The HR detection module **30** is adapted to receive information concerning the random sampled signal **S1,** e.g. value and time stamp when the signal was sampled, and process said received samples in order to calculate an estimation of the HR and/or HRV **S2.** According to an embodiment, the HR detection module **30** calculates an estimation of the HR based on the spectral information of the random sampled signal **S1.** According to an exemplary embodiment, the HR detection module **30** performs least-squares frequency analysis of the random sampled signal **S1** in order to calculate an estimation of the HR. Least-squares spectral analysis (LSSA) or Lomb-Scargle periodogram is a method of estimating a frequency spectrum of unequally spaced data as described, for example, in "Least-squares Frequency Analysis of Unequally Spaced Data", N.R. Lomb, Astrophysics and Space Science 39, 447-462, 1976, in which the power spectral density (PSD) of the samples is calculated using

$$P(\omega) = \frac{1}{2}\left\{ \frac{(\sum x(t_j)\cos\omega(t_j - \tau))^2}{\sum \cos^2\omega(t_j - \tau)} + \frac{(\sum x(t_j)\sin\omega(t_j - \tau))^2}{\sum \sin^2\omega(t_j - \tau)} \right\} \qquad (1)$$

$$\tan(2\omega\tau) = \frac{\sum \sin 2\omega tj}{\sum \cos 2\omega tj} \qquad (2)$$

where x(tj) is the signal at time instant tj (jth sample of the signal) and $\omega$ is the frequency at which the PSD is to be estimated in rad/sec.

According to an exemplary embodiment, the HR detection module **30** calculates the PSD of a plurality of samples of the received randomly sampled signal **S1** and infers the HR information from the same. According to an exemplary embodiment, an average HR over a certain predetermined time interval or window, e.g. 4 seconds, is calculated by finding the frequency (fpk) corresponding to the highest power peak in the PSD of the samples received during that time period or window and then calculating the HR, in beats per minute (bpm), as

$$HR = 60 \times fpk \qquad (3)$$

According to an exemplary embodiment, the HR detection module **30** may calculate the HRV based on the analysis of a plurality of, e.g. at least two, consecutive time windows comprising samples of the randomly sampled signal **S1.** Each window contains the samples received in a certain time period, e.g. 4 seconds, and the HR detection module **30** calculates the spectral density (PSD) of those samples and infers the HR for each window. Then the HRV is calculated as the difference between the HR on said consecutive windows. The plurality of consecutive time windows may be overlapping or non-overlapping time windows.

According to an exemplary embodiment, the system for heart rate detection **100** according to the invention advantageously provides and accurate HR/HRV estimation **S2** from randomly sampled biosignal data **S1** without having to reconstruct an approximation of the original continuous-time biosignal (in the time domain).

According to an exemplary embodiment, the system for heart rate detection **100** according to the invention may be advantageously used for accurate HR/HRV estimation **S2** from randomly sampled PPG data **S1** thereby leading to a low complex, low power pulseoximeter system. It shall be noted that conventional pulseoximeters may employ uniform sampling and estimate the HR and HRV from those acquired (at Nyquist sampling rate) uniform samples. On the other hand, for pulseoximeters employing compressive sampling techniques, current state of the art techniques also rely on a full reconstruction of the original time domain signal in order to perform a subsequent extraction of HR and/or HRV information. Such state of the art approaches are computationally intensive and are not suited for implementation on low power sensor nodes with small form factors. Furthermore, in certain applications, a full reconstruction of the originally sensed biosignal is not required provided the vital information, such as HR/HRV can be extracted, as is explained for the embodiments of the present invention.

According to an exemplary embodiment, the system for heart rate detection **100** according to the invention can advantageously estimate the HR and/or HRV with a high degree of confidence even at very high compression ratios (e.g. greater than 30) of the randomly sampled biosignal data **S1,** where the conventional reconstruction methods fail to reconstruct the signal accurately. It shall be noted that reconstruction techniques based on compressive sampling require a minimum number of samples or measurements in order to be able to faithfully reconstruct the original time domain biosignal. The theory of CS asserts that M out N samples are sufficient for a K-sparse signal for faithful reconstruction, provided M = O(K log (N/K) << N, which puts a limit on the achievable compression ratio (CR = N/M) which guarantees faithful reconstruction. Such limitation is overcome with embodiments of the present invention. It shall be noted that there must be a minimum number of entries in the random sampling matrix in order to be able to estimate the PSD. The boundary on the same depends on the type of biosignal and the required HR/HRV detection accuracy, which can be determined by the person skilled in the art. Additional information can be obtained in "A Prescription for Period Analysis of Unevenly Sampled Time Series", by J. H. Horne and S. L. Baliunas, The Astrophysical Journal, 302: pp. 757-763, March 1986.

The HR estimated according to exemplary embodiments of the invention provides an accuracy of around $\pm 5$ bpm compared to HR estimation based on time domain analysis. HRV calculated according to exemplary embodiments of the invention shows a correlation to HRV estimation based on the time domain signal analysis, with a correlation coefficient greater than 0.95 for compression ratio of 10 and greater than 0.9 for 30 a compression ratio of 30.

According to an exemplary embodiment, the HR detection module **30** may be adapted to search across the entire frequency band of the calculated PSD in order to find the highest power peak frequency (fpk). According to another exemplary embodiment, the HR detection module **30** may be further adapted to search in a narrower frequency band around an average highest power peak frequency (afpk) calculated based on a plurality of windows. According to an exemplary embodiment, the HR detection module **30** may be adapted to calculate the PSD and find the frequency corresponding to the highest power peak (fpk) of four windows of samples and then calculate an average highest power peak frequency (afpk) based on the previous four highest power peak frequencies. Subsequently, for successive windows of samples, the HR detection module **30** may be adapted to just search for the highest PSD power peak frequency (fpk) within $\pm$ 1 Hz of the calculated average highest power peak frequency (afpk). According to an exemplary embodiment, the four windows of samples are consecutive and non-overlapping windows of samples. With this adaptive peak/frequency search scheme, the system advantageously reduces the false estimation of the HR/HRV in presence of motion artifacts, which makes the system more robust and accurate.

According to an exemplary embodiment, the HR detection module **30** may be further adapted to calculate the PSD of the window samples based on values stored in a look-up table. According to an exemplary embodiment, the system for heart rate detection **100** has already knowledge by design of the random sampling instances (tj) and terms $\tau$, $\omega$ and hence $\cos\omega$ ($\tau$ - tj) and $\sin\omega$ ($\tau$ - tj) may be calculated in advance and stored in a look-up table. Said precalculation is based on the fact that the system is expected to work over a known range of HR (30bpm - 300bpm), so the PSD is to be estimated only across a narrow band of frequency ranges from 0.5 Hz to 5 Hz. The frequency resolution is also determined by the desired accuracy in estimating HR, which is typically $\pm 5$ bpm (as per ANSI-AAMI standards), thereby requiring a frequency resolution of 0.08 Hz. Henceforth the frequency interval [0.5, 5] can be divided into linear steps with step size being 0.08 Hz. This technique according to an exemplary embodiment of the invention, advantageously simplifies the computation of the PSD since it eliminates the need for computation of complex trigonometric quantities using CORDIC based methods. This increases the throughput and further reduces complexity and power consumption at the sensor node.

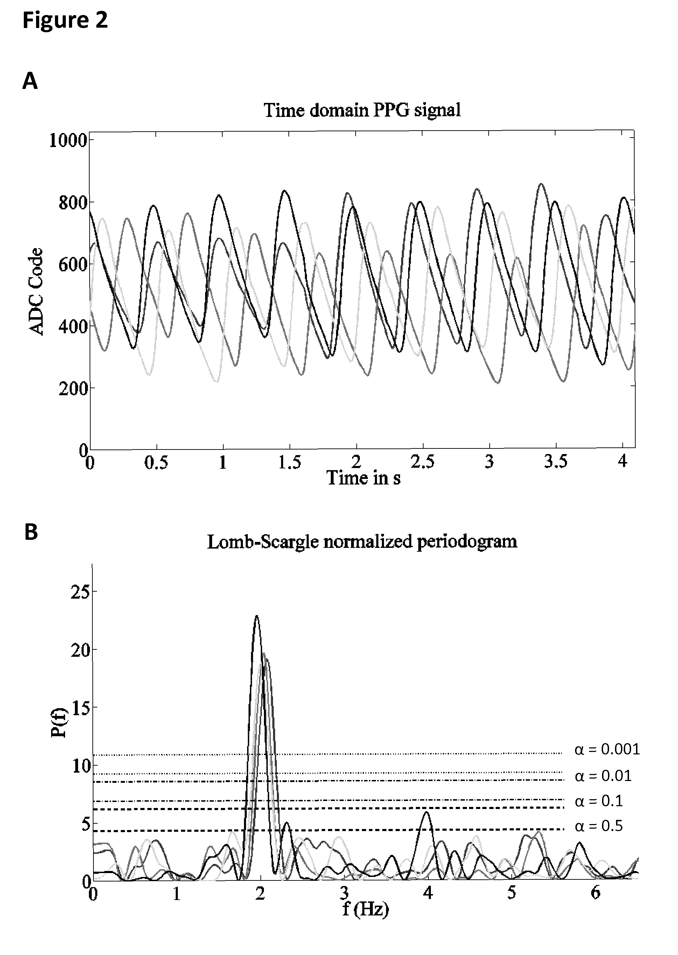

**Figure 2A** shows an exemplary graph of four superimposed time domain PPG signal windows of 4 seconds. Each PPG signal window is part of a time domain PPG signal of 16 seconds that has been sliced into four consecutive 4-second signal windows according to an exemplary embodiment of the invention. **Figure 2B** shows four superimposed PSD calculations of the four PPG signal windows of Figure 2A when said time domain signal windows are randomly sampled at an average rate of 12 Hz (corresponding to a CR of 10). According to an exemplary embodiment, the HR estimation, calculated over each period of 4 seconds, is then calculated by finding the frequency corresponding to the highest peak in the PSD (fpk) and then calculating the HR in bpm as HR = 60 x fpk. It shall be noted that the time period for HR/HRV calculations may vary, e.g. calculation every 5 or 10 seconds, and may be set by the designer or user, depending on the desired implementation.

According to an exemplary embodiment, the HR detection module **30** calculates a HR value based on the PSD of the samples received during a time window of at least 4 seconds. This advantageously represents at least two cycles of a biosignal with a minimum HR of 30bpm (the period being 2 seconds). For a better estimation of the HR more than 2 cycles are desirable. Therefore, longer time windows for HR estimation, e.g. 16 seconds, may be used but this means larger latency and a lower update rate, i.e. the system can provide HR and/or HRV information only every 16 seconds. HRV may be calculated as the difference between the HR calculated in two consecutive windows of figure 2A.

The confidence level in the estimation of the PSD is given by 1-$\alpha$, where $\alpha$ is the probability of estimation being false. It can be observed in Figure 2B that the confidence level in estimation of the PSD is above 99% even at a CR of 10.

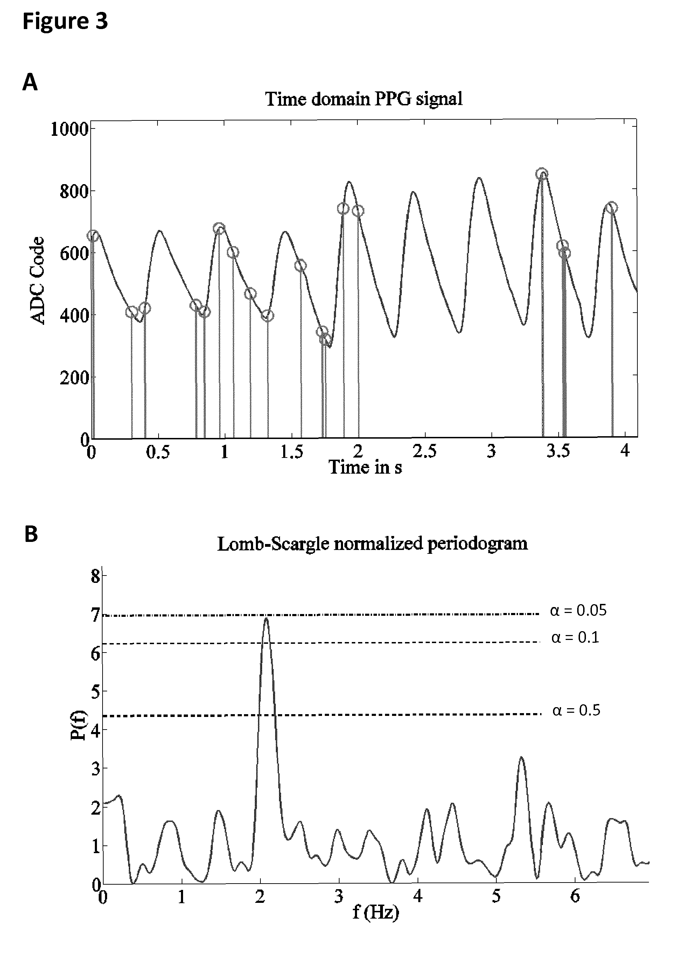

**Figure 3A** shows an exemplary graph of a time domain PPG signal window of 4 seconds, which is randomly sampled according to a CR of 30. **Figure 3B** shows the PSD calculation of the randomly sampled time domain PPG signal window of Figure 3A. It can be seen that there is a predominant peak in the PSD corresponding to the average HR. Although the confidence level in the PSD estimation (95%) is lower than the example of figures 2A and 2B for a CR of 10, it may be still sufficient for certain applications. The described exemplary method for estimation of the HR can then be extended to high compression ratios, e.g. 30, where the conventional methods based on time domain signal reconstruction fail to give satisfactory reconstructed signal quality. Furthermore, according to an exemplary embodiment, the system for heart rate detection **100** can be designed adaptively to achieve a certain trade-off between compression ratio and the desired confidence level in estimation of PSD.

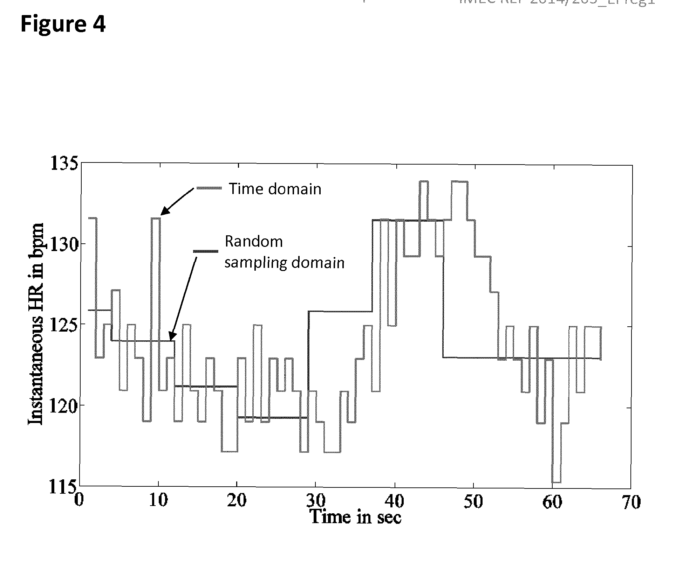

**Figure 4** shows an exemplary graph for comparison of the HRV value as a function of time for an exemplary measured PPG signal, when the HRV is computed using a method according to an embodiment the invention or a conventional time domain method. According to an exemplary embodiment, the original PPG signal is sampled at 125 Hz, while the proposed method of HR calculation is implemented on the original signal after randomly sampling it to obtain a CR of 10, which corresponds to an average sampling rate of 12 Hz. The time window for calculation of the PSD is 4 seconds and the HRV is computed based on non-overlapping windows.

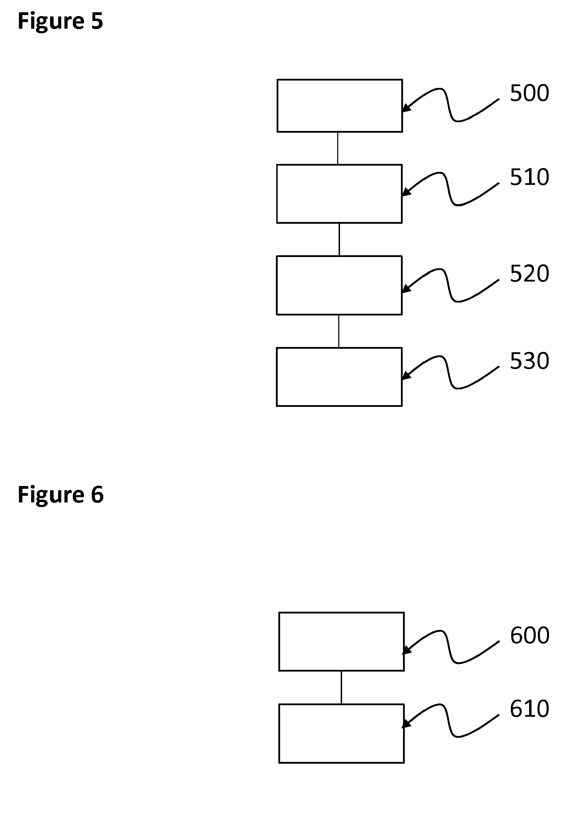

**Figure 5** shows a flow diagram of a method for calculating HR according to an exemplary embodiment of the invention. In **step 500** a plurality of random samples of the biosignal **S1** are acquired during a time interval or window of T seconds. In **step 510** the Lomb-Scargle periodogram is calculated as in equations (1) and (2) (explained with reference to Figure 1). According to an exemplary embodiment, the Lomb-Scargle periodogram is calculated for a frequency range between 0.5 to 5 Hz and with a resolution of 0.08 Hz. In **step 520** a search is done in order to locate the highest power peak of the Lomb-Scargle periodogram and the corresponding frequency (fpk). According to an exemplary embodiment, in the presence of motion artifacts, the search for the highest power peak frequency (fpk) may be limited around $\pm$ 1 Hz of a previously calculated average highest power peak frequency (afpk) based on a plurality of window samples. In **step 530,** the HR is calculated based on the highest power peak frequency (fpk) as in equation (3) (explained with reference to Figure 1). Steps **500** to **530** may be repeated as long as HR information **S2** shall be calculated and provided.

**Figure 6** shows a flow diagram of a method for calculating HRV according to an exemplary embodiment of the invention. In **step 600** HR values are calculated according to the exemplary method shown in Figure 5. Then, in **step 610** a HRV value is calculated as the difference between the HR of two consecutive windows of samples. Step **610** may be repeated as long as HRV information **S2** shall be calculated and provided.

It shall be noted that the HR detection module **30** may be implemented according to hardware and/or software state of the art techniques, comprising for example a microprocessor, microcontroller or digital signal processor that can understand and execute software program instructions. Some programmable hardware logic and memory means may be specifically designed also for executing the method or parts of it according to exemplary embodiments of the invention.

**Claims**

**1.** A system (100) for heart rate detection comprising:

a random sampling module (10) configured for providing nonuniform random samples (S1) below Nyquist rate of a biosignal that contains heart rate information; and
a heart rate detection module (30) configured for receiving a plurality of said nonuniform random samples (S1) during a predetermined time window and calculating a heart rate value (S2) based on the received time window samples;

**characterized in that**
the heart rate detection module (30) is configured to calculate a power spectral density based on a Lomb-Scargle periodogram of said window samples and calculate the heart rate value (S2) based on the frequency corresponding to the highest power peak of the calculated power spectral density.

**2.** A system (100) for heart rate detection according to claim 1 wherein the heart rate detection module (30) is configured to allocate the received nonuniform random samples (S1) into a plurality of windows of samples and calculate the heart rate value (S2) for that plurality of window samples.

3. A system (100) for heart rate detection according to any preceding claim wherein the heart rate detection module (30) is configured to search for the highest power peak of the calculated power spectral density of a window of samples within a limited frequency range around an average highest power peak frequency calculated based on a plurality of windows.

4. A system (100) for heart rate detection according to any preceding claim wherein the heart rate detection module (30) is configured to calculate the power spectral density of a window of samples with the use of data stored in a look-up table, said data representing a trigonometric calculation.

5. A system (100) for heart rate detection according to any preceding claim wherein the heart rate detection module (30) is configured to calculate the power spectral density of a window of samples in a frequency band between 0.5 and 5Hz and with a frequency resolution of 0.08 Hz.

6. A system (100) for heart rate detection according to any preceding claim wherein the window of samples is a time window of at least 4 seconds.

7. A system (100) for heart rate detection according to any preceding claim wherein the heart rate detection module (30) is further configured to calculate a heart rate variability value (S2) as the difference between the heart rate value of two consecutive windows of samples.

8. A system (100) for heart rate detection according to any preceding claim wherein the biosignal that contains heart rate information is an ECG, BCG or PPG signal.

9. Electronic device comprising an ECG, BCG or Pulseoximetry system (100) for heart rate detection according to any of the preceding claims.

10. A method for heart rate detection comprising:

receiving a plurality of nonuniform random samples below Nyquist rate of a biosignal that contains heart rate information (500);
calculating a power spectral density based on a Lomb-Scargle periodogram of said received plurality of samples (510);
finding a frequency corresponding to the highest power peak of the calculated power spectral density (520); and
calculating a heart rate value based on said found highest peak power frequency (530).

11. A method for heart rate detection according to claim 10 further comprising:

allocating the plurality of received nonuniform random samples into a plurality of windows of samples and calculating the heart rate value for at least two consecutive windows of samples (600); and
calculating a heart rate variability value as the difference between the heart rate value of said two consecutive windows of samples (610).

12. A computer program product comprising computer program code means adapted to calculate a heart rate of heart rate variability (S2) according to the method of claims 10 or 11 when said program is run on a computer.

13. A computer readable storage medium comprising a computer program according to claim 12.

**Figure 1**

<u>100</u>

| 10 | S1 → | 20 | → | 30 | S2 → |

# Figure 2

**A**

Time domain PPG signal

**B**

Lomb-Scargle normalized periodogram

## Figure 3

**A**

Time domain PPG signal

**B**

Lomb-Scargle normalized periodogram

Figure 4

**Figure 5**

500

510

520

530

**Figure 6**

600

610

Europäisches
Patentamt

European
Patent Office

Office européen
des brevets

# EUROPEAN SEARCH REPORT

Application Number

EP 15 15 1890

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2009/216137 A1 (HOLLAND ALEXANDER [US]) 27 August 2009 (2009-08-27) * paragraphs [0007], [0028] - [0029], [0053] - [0056], [0058], [0071], [0075] - [0077] * * figure 12 * | 1-13 | INV. A61B5/024 A61B5/00 |
| X | PABLO LAGUNA ET AL: "Power Spectral Density of Unevenly Sampled Data by Least-Square Analysis: Performance and Application to Heart Rate Signals", IEEE TRANSACTIONS ON BIOMEDICAL ENGINEERING, IEEE SERVICE CENTER, PISCATAWAY, NJ, USA, vol. 45, no. 6, 1 June 1998 (1998-06-01), XP011006554, ISSN: 0018-9294 * pages 698, 707 * | 1-13 | |
| X | LAGUNA P ET AL: "Power spectral density of unevenly sampled heart rate data", ENGINEERING IN MEDICINE AND BIOLOGY SOCIETY, 1995., IEEE 17TH ANNUAL C ONFERENCE MONTREAL, QUE., CANADA 20-23 SEPT. 1995, NEW YORK, NY, USA,IEEE, US, vol. 1, 20 September 1995 (1995-09-20), pages 157-158, XP010215377, DOI: 10.1109/IEMBS.1995.575048 ISBN: 978-0-7803-2475-6 * pages 157-158 * | 1-13 | **TECHNICAL FIELDS SEARCHED (IPC)** A61B |

-/--

The present search report has been drawn up for all claims

| Place of search | Date of completion of the search | Examiner |
|---|---|---|
| The Hague | 18 April 2016 | Vanderperren, Yves |

CATEGORY OF CITED DOCUMENTS

X : particularly relevant if taken alone
Y : particularly relevant if combined with another
 document of the same category
A : technological background
O : non-written disclosure
P : intermediate document

T : theory or principle underlying the invention
E : earlier patent document, but published on, or
 after the filing date
D : document cited in the application
L : document cited for other reasons

& : member of the same patent family, corresponding
 document

EPO FORM 1503 03.82 (P04C01)

Europäisches
Patentamt

European
Patent Office

Office européen
des brevets

## EUROPEAN SEARCH REPORT

Application Number

EP 15 15 1890

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LIU XIAO-FANG ET AL: "Study on Algorithm for Spectral Analysis of Instantaneous Heart Rate", BIOINFORMATICS AND BIOMEDICAL ENGINEERING, 2008. ICBBE 2008. THE 2ND INTERNATIONAL CONFERENCE ON, IEEE, PISCATAWAY, NJ, USA, 16 May 2008 (2008-05-16), pages 2107-2110, XP031267742, ISBN: 978-1-4244-1747-6 * pages 2017-201 * | 1-13 | |
| A | CHEN SZI-WEN ET AL: "Compressed Sensing Technology-Based Spectral Estimation of Heart Rate Variability Using the Integral Pulse Frequency Modulation Model", IEEE JOURNAL OF BIOMEDICAL AND HEALTH INFORMATICS, IEEE, PISCATAWAY, NJ, USA, vol. 18, no. 3, 1 May 2014 (2014-05-01), pages 1081-1090, XP011547002, ISSN: 2168-2194, DOI: 10.1109/JBHI.2013.2282307 [retrieved on 2014-05-01] * pages 1081, 10 * | 7,11 | |

TECHNICAL FIELDS
SEARCHED       (IPC)

The present search report has been drawn up for all claims

| Place of search | Date of completion of the search | Examiner |
|---|---|---|
| The Hague | 18 April 2016 | Vanderperren, Yves |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 15 15 1890

This annex lists the patent family members relating to the patent documents cited in the above-mentioned European search report.
The members are as contained in the European Patent Office EDP file on
The European Patent Office is in no way liable for these particulars which are merely given for the purpose of information.

18-04-2016

| Patent document cited in search report | Publication date | Patent family member(s) | Publication date |
|---|---|---|---|
| US 2009216137 A1 | 27-08-2009 | US 2009216137 A1<br>WO 2009108549 A2 | 27-08-2009<br>03-09-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **E. J. CANDÈS et al.** An Introduction to compressive sampling. *IEEE Signal Processing Magazine,* March 2008, vol. 25, 21-30
- **A.M.R. DIXON et al.** Compressed Sensing System Considerations for ECG and EMG Wireless Biosensors. *IEEE Transactions on Biomedical Circuits and Systems,* April 2012, vol. 6 (2
- **H. GARUDARI et al.** Artifacts Mitigation in Ambulatory ECG Telemetry. *Proc. IEEE Int. Conf. e-Health Networking Applications and Services,* 2010, 338-344
- **P.K. BAHETI et al.** An ultra low power pulse oximetersensorbased on compressed sensing. *Body Sensor Networks,* 2009, 144-148

- **J. ROMBERG.** Compressive Sensing by Random Convolution. *SIAM Journal on Imaging Sciences,* October 2009, vol. 2 (4
- **E. CANDES ; J. ROMBERG.** Sparsity and Incoherence in Compressive Sampling. *Inverse Prob.,* 2007, vol. 23 (3), 969-985
- **N.R. LOMB.** Least-squares Frequency Analysis of Unequally Spaced Data. *Astrophysics and Space Science,* 1976, vol. 39, 447-462
- **J. H. HORNE ; S. L. BALIUNAS.** A Prescription for Period Analysis of Unevenly Sampled Time Series. *The Astrophysical Journal,* March 1986, vol. 302, 757-763